# EUROPEAN PATENT APPLICATION

(11) **EP 1 785 087 A1**
(43) Date of publication of application: **16.05.2007**
(21) Application number: 05110693.8
(22) Date of filing: 14.11.2005
(51) Int. Cl.: A61B 5/02

(54) **Cardiovascular analysis system and method**

(71) Applicant: Microlife Intellectual Property GmbH, 9443 Widnau (CH)
(72) Inventor: Asmar, Roland, 2, rue du Docteur Blanche 75016 Paris (FR)
(74) Representative: Müller, Christoph Emanuel

(57) **Abstract**

A cardiovascular analysis system comprises a first blood pressure measuring cuff adapted to be placed at the first measurement site and a second blood pressure measuring cuff adapted to be placed at a second measurement site of a subject. A measuring device is connected to or connectable to these cuffs (10, 20) for determining first and second blood pressure values. The device is further provided with means (2) for determining a relationship value between the first blood pressure and the second blood pressure values. The system is operatable in a monitoring mode, where the relationship value is repeatedly monitored.

## Description

The invention relates to a cardiovascular analysis system and to a method for cardiovascular analysis. Arteriosclerotic cardiovascular diseases represent a major health problem. For determining arteriosclerosis, it is known that measuring the so called superior-and-inferior-limb blood pressure index can be used as an indication of inferior limb arterial diseases and as an atherosclerosis index. This index is often calculated as the ratio between a systolic blood pressure of an ankle as an example of an inferior limb blood pressure and of a systolic blood pressure of an upper arm as a superior limb blood pressure. This index is also called the ankle-arm blood pressure index and is sometimes referred to as AAI, API or ABI. A system for measuring such ABI is e.g. disclosed in EP 1 050 267 A1.

Known systems have, however, certain drawbacks. In particular, information provided by such systems is often not sufficient for a doctor to make a diagnosis. In fact, most of the available systems are a bit complicated to use and therefore mainly reserved to specialists or research labs. Moreover, their measurements provide values based on one or very few measurements without any information on these indexes variability.

It is therefore an object of the present invention to overcome the drawbacks of the prior art, in particular to provide a cardiovascular analysis system and method which allow to give the doctor sufficient information as a basis for a reliable diagnosis in view of cardiovascular diseases, in particular in view of arteriosclerosis. A further object of the invention is to provide a system which also allows to verify the effectiveness of a treatment against such diseases.

These and other objects are solved with a system and a method according to the features of the independent patent claims.

The cardiovascular analysis system according to the invention is in particular used for arteriostenosis detection and for diagnosing a patient in view of arteriosclerosis. In fact, the system allows verification of the normal distribution of blood pressure in different parts of the human body; allows atherosclerosis detection and monitors its variation under treatment. Furthermore, the system provides major information given by the index variability which allows physicians to evaluate the arterial system but also to assess the patho-physiology of major cardiovascular function and diseases such as baroreceptors.

The system comprises at least a first blood pressure measuring cuff. This first cuff is adapted to be placed at a first measuring site of an individual. In particular, this first measurement site is an upper arm, in particular the right upper arm of the patient. The system further comprises at least a second blood pressure measuring cuff which is adapted to be placed at a second measurement site of the subject. The second measurement site may be the left upper arm or an ankle. A measuring device is connected to or connectable to the first and the second cuff. The measuring device is designed for determination of first and second blood pressure values which are measured at the first and at the second measurement site. The system further comprises means for determining a relationship value between the first and the second blood pressure value. In particular, such a relationship value may be the ratio between the systolic and diastolic blood pressure values measured at the first and at the second measurement site. In the case of measurements at the left and right upper arm, this ratio can be especially used in order to evaluate the differences and assess the presence or not of any anisotensia (difference between the 2 sites at the left and right upper arms).
However, it is also possible to determine more complicated relationships than a ratio based on appropriate clinical findings.

According to the invention, the system is operable in a monitoring mode. In this monitoring mode, the relationship value is repeatedly measured. In context of the present invention, monitoring means a repeated determination of the relationship value at a predetermined time interval. In the monitoring mode, determination of the relationship value is e.g. repeated for a predetermined number of times, for a predetermined duration or also until the measurement device is stopped.

The relationship values measured in the monitoring mode are typically stored in a memory in the system. A care person, in particular a doctor, may refer to these data and analyse the relationship value varying in the course of time. In particular, variations of the relationship value may give the doctor an indication of specific health problems.

For this purpose, according to a preferred embodiment of the invention, the system further is provided with means for determining statistical or variability information relating to the relationship value. In particular, the system may be provided with means for determining the mean value, the standard deviation and/or the variance of the relationship value over a certain number of subsequent measurements. In fact, information provided by the mean values, the standard deviation and the variation coefficients have different physiological and patho-physiological significance. Therefore, the mean values give information about blood pressure and the arterial status, the standard deviation provides information on the blood pressure variability and the variation coefficient gives normalized variability. Assessment of these parameters together with those of the heart rate allows physician to evaluate the arterial functions such as the baroreceptor sensitivity and function. Such statistical information may be directly calculated in the system and displayed on a display of the system. The system thus provides the care person with statistical information of the relationship value which may be an indication of specific health problems.

Typically, the system is provided with a display on which the first and the second blood pressure values and the relationship value and optionally pulse pressure values are displayed after each measurement. Furthermore, there may be a display for the statistical information of some or all the measured parameters. The calculation may consider also the heart rate values. A simple linear correction coefficient between the relationship value and the heart rate can be provided.

Furthermore, parameters such as the heart rate or the correlation between the heart rate and the blood pressure mean and indexes values may be displayed.

The display may have a plurality of display areas on which the different values based on different measurement sites may be displayed in parallel.

According to a preferred embodiment of the invention, the measuring device is arranged within one single housing and the first and the second cuff may be connected in parallel to the measuring device. In the housing, all processing and calculation means for determining the relationship value and the statistical information may be arranged. A device which is easy to be used is provided.

It is, however, also possible to provide two separate blood pressure measuring devices adapted for measurement at the first and at the second measurement site and to transfer blood pressure values determined by these devices to a separate unit where the relationship value and optionally statistical information will be determined.

According to a preferred embodiment of the invention, the system is operatable in at least two measurement modes. These modes typically may be a single measurement mode, a quick measurement mode and an averaging measurement mode. In the single measurement mode, each blood pressure result is derived from one single inflation and deflation cycle of the first and of the second cuff. In the quick measurement mode, one single measurement is also taken. However, the inflation and deflation cycle is done quickly, e.g. shorter than 30 seconds, depending on the patient's blood pressure values. Such a quick mode has greater comfort to the patient. However, measurement results may be somewhat less accurate. In the averaging measurement mode, each blood pressure result is derived as an average of a series of inflation and deflation cycles. Typically, one or three measurement cycles may be made. Averaging measurement mode needs more time but it is preferable because it provides more accurate measurement results.

The system may be provided with means for switching the operation between these measurement modes. Depending on the requirements for a specific measurement and depending on the patient's individual preferences in view of duration of a measurement and comfort, appropriate measurement modes may be selected.

According to a further preferred embodiment of the invention, the system is not only operatable in the continuous monitoring mode as mentioned above. It may also be operated in an automatic measurement mode and/or a manual measurement mode. In the manual measurement mode, the system will be used in a traditional manner for determination of blood pressure via a stethoscope. In the manual mode, the system thus only inflates and deflates the first cuff placed around the first measurement site and displays current blood pressure values measured within the cuff. This measurement mode allows the doctor to verify the accuracy of measurements on the basis of reliable traditional measurements. This manual mode is particular useful for a physician for patients for whom automatic oscillometric method may be less accurate such as in presence of arrhythmia. In the automatic measurement mode, the system is automatically determining the blood pressure values on one or on both cuffs. In this operating mode, the system works in a manner comparable to traditional automatic blood pressure measuring devices without repeating the measurements.

The system may also be provided with means for switching between this continuous monitoring mode and the automatic and/or the manual measurement mode.

According to a further aspect of the invention, there is provided a method for cardiovascular analysis. In particular, this method is carried out with a system as described herein above. In a first step, first blood pressure values are determined at a first measurement site of an individual. In another step, second blood pressure values are determined at a second measurement site of the individual. Based on these blood pressure values, a relationship value is determined.

The steps of determining the first and second blood pressure values and forming the relationship value are repeated during a predetermined or a predeterminable period of time or a number of times.

Preferably, in a further step, statistical or variability information of at least this relationship value are determined. In particular, mean values, the variance or the standard deviation of the relationship values may be calculated. While it is preferred to determine these values in a system as described herein above, it is also conceivable to determine these values manually on the basis of data stored in such a system.

The invention will now be explained in more detail with reference to the following embodiments and the accompanying drawings which show:
- Figure 1: A schematic view of a system according to the invention
- Figure 2: A schematic overview of a control area of a measuring device according to the invention
- Figure 3: An example of a display of a system according to the invention
- Figure 4: A schematic representation of the different components of a measuring device according to the invention

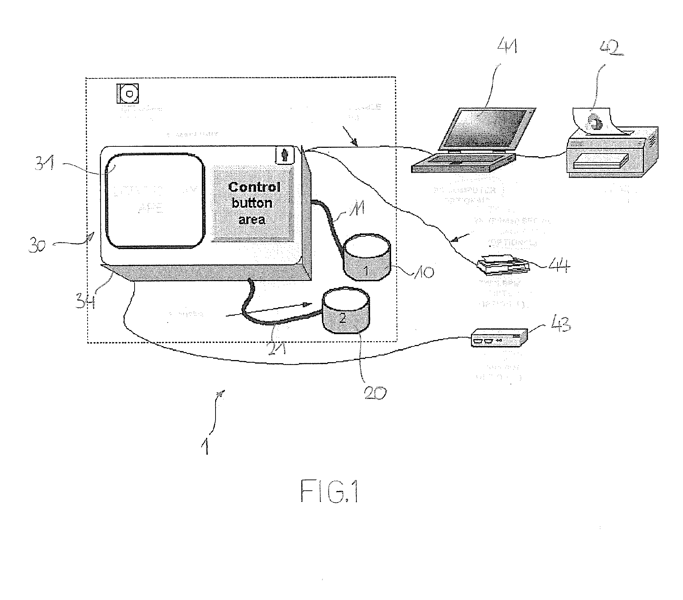

Figure 1 schematically shows a system 1 embodying the features of the present invention. The system 1 basically consists of a housing 34 of a measuring device 30. A first cuff 10 and a second cuff 20 are attached to the measuring device 34 by means of tubes 11, 21, respectively. The measuring device 30 is provided with a display 31 arranged on a surface of the housing 34.

The system 1 according to the invention may further be optionally provided with a personal computer 41, with a printer 42 attached to the personal computer, with a power supply 43 for operating the system 1 and/or with a printer 44 directly connected to the measuring device 30. Wire connections are shown in Figure 1. This may be typically USB or RS232 connections. However, wireless data transmission may also be possible.

When in operation, the system 1 according to the invention is used to measure a blood pressure of an individual on the basis of the pressure measured in the cuff 10 applied at the upper arm of the patient and to measure a second blood pressure value on the basis of the cuff 20 applied to the ankle of the patient. Determination of these blood pressures is made in a known manner, in particular on the basis of the oscillometric method.

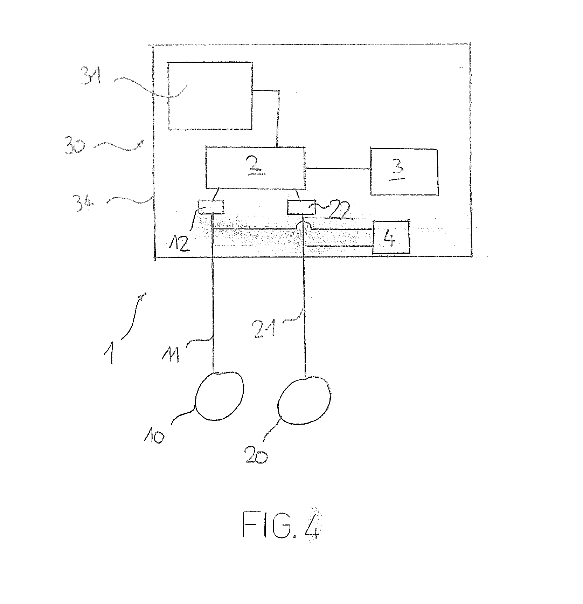

For this purpose (see also Figure 4) the measuring device 30 is provided with standard components contained in a blood pressure measuring device.

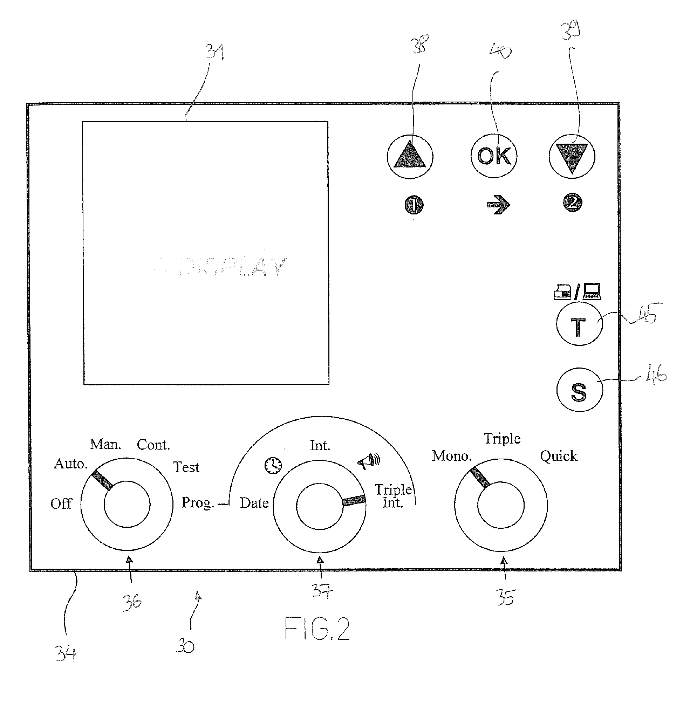

The system 1, in particular the measuring device 30 is provided with a plurality of operation buttons or switches. Figure 2 schematically shows a control area of the measuring device 30 which is arranged within the housing 34. A display 31 is arranged in the housing 34. A first measurement mode switch 35 allows to switch operation between a single or mono mode, a triple or average mode and a quick measurement mode.

A switch 36 allows to switch between a plurality of operating modes, in particular between an automatic, a manual and a continuous measurement mode. Furthermore, switch 36 may be used to start a test mode, to turn the measuring device 30 off and to enter program instructions.

A set up switch 37 is used to set specific parameters of the measuring device 30 as will be explained hereinafter.

In the single measurement mode, a blood pressure measurement is made in a conventional manner, e.g. by inflating and deflating the cuff 10 and/or the cuff 20 while taking pressure measurements during the deflation period. The duration of inflation and deflation typically is 1 Minute. In the triple measurement mode, the measurement cycle as described above is repeated three times. Subsequently, averages of the systolic and diastolic blood pressure values determined during these three measurements will be formed. In the quick measurement mode, inflation and deflation is made more quickly, e.g. during 30 seconds.

The operation of the device further will be defined by the setting of the switch 36. If the switch 36 is in the "Auto" operation mode, one blood pressure measurement will be automatically taken. Depending on the setting of the button 35, one or three measurement cycles will be made and a final result of systolic and diastolic blood pressure will be determined and displayed. This automatic, single measurement may be made on one cuff or on both cuffs 10, 20.

In the manual operation mode, the device 30 only inflates and deflates the first cuff 10. The operator, in particular a doctor may do manual systolic and diastolic blood pressure measurements by means of a stethoscope. In this operating process, current pressure values in the cuff will be displayed on the display 31. However, no automatic calculation or determination of blood pressure values will be made.

In the continuous operation mode, the device 30 is making continuous blood pressure measurements. Measurements are repeated at a predetermined time interval. Measurements can be done on one cuff or on both of the cuffs 10, 20.

If the switch 36 is in the test position, the unit can be calibrated or can be checked with the help of a static reference pressure device.

The switch 36 can further be placed in a program position. In the program position, parameters of the device 30 such as date/time or intervals can be programmed.

The set up switch 37 is used for defining process parameters of the device 30 if the switch 36 is in the program position. In particular, the date and time can be entered. The duration of an interval between subsequent measurements in the continuous mode can be entered if the set up switch 37 is in the position "Int" The interval between subsequent measurements for average measurement in the triple mode can be entered if the set up switch 37 is in the position "Triple Int". Furthermore, an alarm time may be set if the set switch 37 is in the alarm position.

Push buttons 38, 39 are used for decreasing or increasing parameter values such as date, time or intervals. A confirmation button 40 is used to confirm entered values. These values can be entered if the switch 36 is in the program mode. If the switch 36 is in one of the automatic, manual or continuous modes, the buttons 38, 39 are used to start measurement cycles on one or on both of the cuffs 10, 20.

A communication push button 45 is used to transfer data to a serial port (not shown) for transfer of data to a peripheral device such as a personal computer or a printer. A pump control push button 46 is used to manually start inflation or deflation of the currently used cuff.

As will be shown hereinafter in more detail, the device 30 according to the present invention is particularly suitable to determine a relationship value between blood pressure values measured with the first cuff 10 and the second cuff 20. For this purpose, a measurement cycle is started by pressing in parallel the push button 38 and push button 39 when the switch 36 is either in the automatic or in the continuous mode.

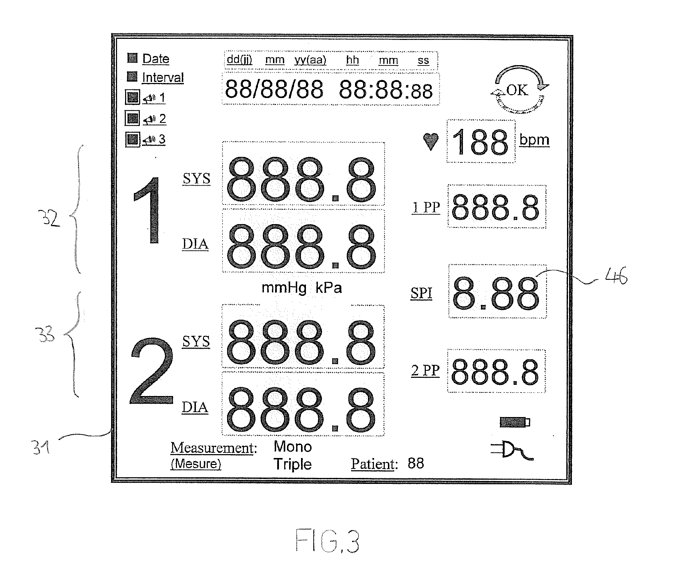

Figure 3 schematically shows the display 31. The display 31 is basically divided into a first display area 32 and into a second display area 33. In the first display area 32 systolic and diastolic blood pressure values measured with the first cuff 10 will be displayed. In the second display area 33, systolic and diastolic blood pressure values measured with the second cuff 2 are displayed. In the first and second display area 32, 33, furthermore, pulse pressure (i.e. the difference between the systolic and the diastolic blood pressure value) are displayed in the area labelled with 1 PP and 2 PP, respectively. In an index display area 46, the ratio between the systolic values measured by the first cuff 10 and by the second cuff 20 are displayed.

The device can be used for several patients. In this case, the number of patient may be counted and indicated.

The display 31 further has additional display areas for display of battery status, charging mode, date, time or for operation status which are of no particular importance in view of the concept of the present invention and which are not explained in more detail.

Figure 4 shows a schematic view of a system 1 according to the invention. The same reference numerals as in the Figures 1 to 3 designate the same parts. The cuffs 10, 20 are attached via tubes 11, 21 to the housing 34 of the measuring device. Operation of the device is controlled by a microprocessor 2. Attached to the tubes 11, 21 there are pressure measuring sensors 12, 22 for measuring the pressure inside the cuff. The measurement sensors 12, 22 are connected to inputs of the microprocessor 2 for transfer of pressure data. Appropriate components such as analogue/digital converters or amplifiers generally known to those skilled in the art may be used.

Preferably two pumps 4 are used for inflating the cuffs 10, 20 to a predetermined pressure. After the cuffs have been put around the measurement extremities, the cuffs are pressurised by operation of the two pumps. Each cuff is pressurized individually.

The pressure in the cuffs is then released with a pre-defined deflation rate of e.g. 4mmHg/s (in the normal measurement mode) by opening the deflation valves (not shown). The deflation valves can be either passive linear valves with a fixed deflation rate, or electronically controlled linear valves. During release of the pressure, the pressure within the cuffs 10, 20 is continuously measured by the sensors 12, 22. Based on these values, the microprocessor 2 determines systolic and diastolic blood pressure values for each of the cuffs 10, 20 according to known algorithms of the oscillometric measurement principle. The measurement values are stored in a memory 3 for subsequent use. The microprocessor 2 further calculates a ratio between the systolic values determined on the basis of the pressure in the first cuff 10 and on the basis of the second cuff 20. This ratio is also stored in the memory 3.

The microprocessor 2 is coupled to a display 31 for display of the blood pressure values and the index or ratio between the values measured with the first cuff 10 and the second cuff 20. If the system 1 is used in a monitoring mode, measurements are repeated and the values of a plurality of subsequent measurements, in particular blood pressure values and the index, are stored in the memory 3. Based on the series of measurement values, the microprocessor 2 calculates statistical data, in particular a mean value of the ratio between the systolic blood pressure values and a standard deviation thereof and displays these data in the display 31. Alternatively, it is also possible to transfer the data stored in the memory 3 to an external device such as a personal computer through a communication interface (not shown).

The device is further provided with operation buttons (see Figure 3) and a power supply which is not shown in detail in Figure 4. Optionally, the device can also be operated by internal batteries.

Typically, the interval between subsequent measurements in the monitoring mode may be set between 30 seconds and 99 minutes and measurements may be repeated for a predetermined time. Measurements may also be repeated continuously until the device will be stopped.

By a way of example, a monitoring cycle based on seven subsequent measurements taken at an interval of e.g. 1 minute will be shown in table 1. In seven measurements, measurement values of a first cuff, applied to the arm of a patient and of a second cuff, applied to the ankle of the patient are determined. Table 1 shows the systolic blood pressure, the diastolic blood pressure, the heart rate and the pulse pressure (defined as systolic - diastolic) measured by each cuff in each of the measurements.

In particular, pulse pressure gives a further indication in view of arterial rigidity and arterial stiffness.

With such a monitoring mode it will be possible to alert a physician if blood pressures are too low or too high, e.g. as compared to thresholds set up by the physician. In the case of too high or too low pressures, an alarm may start ringing.

Statistical values typically will be displayed on the screen at the end of a measurement session. Alternatively or in addition, the statistical values could also be printed on the printer. Typically, calculation will be made by software programs integrated within the device for subsequent data exportation. It is, however, also possible to use external calculation algorithms.

**Table 1**

| An example for the calculation in-dexes | | | | | | |
|---|---|---|---|---|---|---|
| | | | | | | |
| | | **Cuff 1** | Arm | | | |
| | Time | SBP | DBP | MBP | PP | HR |
| Measure 1 | | 142 | 96 | | 46 | 78 |
| 2 | | 138 | 88 | | 50 | 68 |
| 3 | | 125 | 102 | | 23 | 56 |
| 4 | | 145 | 85 | | 60 | 80 |
| 5 | | 163 | 78 | | 85 | 84 |
| 6 | | 125 | 90 | | 35 | 58 |
| 7 | | 145 | 88 | | 57 | 68 |
| | | | | | | |
| Means Values | | 140 | 90 | | 51 | 70 |
| SD | | 9,51 | 5,51 | | 14,12 | 8,90 |
| V coefficient= SD/Mean x100 | | 6,77 | | | | |
| | | | | | | |
| | | | | | | |
| | | | | | | |

| | | **Cuff 2** | Ankle | | | |
|---|---|---|---|---|---|---|
| | Time | SBP | DBP | MBP | PP | HR |
| Measure 1 | | 152 | 95 | | 57 | 78 |
| 2 | | 145 | 86 | | 59 | 68 |
| 3 | | 138 | 100 | | 38 | 56 |
| 4 | | 148 | 80 | | 68 | 80 |
| 5 | | 170 | 74 | | 96 | 84 |
| 6 | | 140 | 85 | | 55 | 58 |
| 7 | | 154 | 84 | | 70 | 68 |
| | | | | | | |
| Means Values | | 150 | 86 | | 63 | 70 |
| SD | | 7,80 | 6,41 | | 12,61 | 8,90 |
| V coefficient | | 5,21 | | | | |
| | | | | | | |
| | | | | | | |

| Index | | **Indexes** | | | | |
|---|---|---|---|---|---|---|
| Measure 1 | | 1,07 | | | | |
| 2 | | 1,05 | | | | |
| 3 | | 1,10 | | | | |
| 4 | | 1,02 | | | | |
| 5 | | 1,04 | | | | |
| 6 | | 1,12 | | | | |
| 7 | | 1,06 | | | | |
| | | | | | | |
| Means Values | | 1,07 | | | | |
| SD | | 0,03 | | | | |
| V coefficient | | 2,48 | | | | |

Mean values, standard deviations and a variability coefficient (defined at the ratio between the standard deviation and the mean value as a percentage) will be calculated for each of the measurement values on the basis of the seven measurements for each cuff. Furthermore, index values, i.e. the ratio between the systolic blood pressure of the second cuff at the ankle and of the first cuff at the arm will be determined. In accordance with the embodiment shown in the table, this index varies between 1,02 in the first measurement and 1,12 in the sixth measurement. For these index values, also mean values, standard deviations and variability coefficient is calculated.

The evaluation of the mean value informs the doctor whether the measurement fit is normal or not. The variability indexes indicate whether there is any improvement of the variability which could be related to the arterial structure or function.

## Claims

1. A cardiovascular analysis system (1), in particular for arteriostenosis inspection, the system comprising
at least a first blood pressure measuring cuff (10) adapted to be placed at a first measurement site of a living subject,
at least a second blood pressure measuring cuff (20) adapted to be placed at a second measurement site of said subject,
a measuring device (30) connected or connectable to said first and second cuff (10, 20) for determining first blood pressure values measured at said first measurement site and for determining second blood pressure values measured at said second measurement site and
means (2) for determining a relationship value between said first blood pressure values and said second blood pressure values,
**characterised in that** said system is operatable in a monitoring mode, where said relationship value is repeatedly monitored.

2. A system according to claim 1, wherein said system further comprises means for determining statistical and/or variability information relating to said relationship value, in particular information selected from the mean value, standard deviation and variance.

3. A system according to one of the claims 1 or 2 wherein said relationship value is the ratio between the systolic blood pressure measured at said first and measured at said second measurement site.

4. A system according to one of the claims 1 to 3, wherein said measuring device (30) is provided with a display (31) for displaying at least one of the following values
- first and second blood pressure values;
- relationship values;
- pulse pressure values;
- heart rate;
- the correlation between heart rate and the blood pressure mean and indexes values.

5. A system according to claim 4, wherein said display has a plurality of display areas (32, 33) for concurrently displaying said values.

6. A system according to one of the claims 1 to 5, wherein said measuring device (30) is arranged within one single housing (34).

7. A system according to one of the claims 1 to 6, wherein the system is operatable in at least two modes selected from the group of a single measurement mode, a quick measurement mode and an averaging measurement mode.

8. A system according to claim 7, wherein the system comprises means (35) for switching operation between said measurement modes.

9. A system according to one of the claims 1 to 8, wherein the system is operatable in a continuous monitoring mode and in at least one of a automatic measurement mode and a manual measurement mode.

10. A system according to claim 9, wherein said system comprises means (30) for switching between said continuous monitoring mode, said automatic measurement mode and/or said manual measurement mode.

11. A method for cardiovascular analysis, in particular for arteriostenosis analysis, in particular with a system (1) according to one of the claims 1 to 10, the method comprising the steps of
a) Determining first blood pressure values at a first measurement site of an individual
b) Determining second blood pressure values at a second measurement site of this individual
c) Determining a relationship value between said first and second blood pressure values
d) Repeating steps a) to c) at least once after a predetermined or predeterminable period of time.

12. A method according to claim 11, comprising the further step of determining statistical or variability information of said relationship value, in particular determining mean values, variance or standard deviation of said relationship values.
